Europäisches Patentamt

European Patent Office

Office européen des brevets

(19)

(11) Veröffentlichungsnummer: **0 220 549 B1**

(12) **EUROPÄISCHE PATENTSCHRIFT**

(45) Veröffentlichungstag der Patentschrift: **20.02.91**

(51) Int. Cl.⁵: **A61N 1/36**, A61B 5/00

(21) Anmeldenummer: **86113803.0**

(22) Anmeldetag: **06.10.86**

(54) Messvorrichtung zur intrakardialen Erfassung der Blutsauerstoffsättigung.

(30) Priorität: **17.10.85 DE 3537017**
**16.05.86 DE 3616524**

(43) Veröffentlichungstag der Anmeldung:
**06.05.87 Patentblatt 87/19**

(45) Bekanntmachung des Hinweises auf die
Patenterteilung:
**20.02.91 Patentblatt 91/08**

(84) Benannte Vertragsstaaten:
**DE FR GB IT NL SE**

(56) Entgegenhaltungen:
**DE-A- 3 222 279**

(73) Patentinhaber: **Siemens Aktiengesellschaft**
**Wittelsbacherplatz 2**
**D-8000 München 2(DE)DE FR GB IT NL**

Patentinhaber: **Siemens Elema AB**
**Röntgenvägen 2**
**S-171 95 Solna 1(SE)SE**

(72) Erfinder: **Liess, Hans-Dieter, Prof. Dr.**
**Pfaffenkamer Strasse 5**
**D-8193 Münsing(DE)**
Erfinder: **Heinze, Roland**
**Simbacher Strasse 9**
**D-8000 München 80(DE)**

**Beschreibung**

Die Erfindung betrifft eine Meßvorrichtung zur intrakardialen Erfassung der Blutsauerstoffsättigung zur Frequenzregelung eines Herzschrittmachers mit einer Meßsonde, die einen Lichtsender und einen Lichtempfänger enthält, wobei der Lichtempfänger das vom Lichtsender ausgesandte und vom Blut reflektierte Licht empfängt und wobei der Lichtsender und der Lichtempfänger über zwei gemeinsame Leitungen an eine Auswerteschaltung angeschlossen sind, die die Meßsonde mit einem konstanten Strom oder einer konstanten Spannung beaufschlagt.

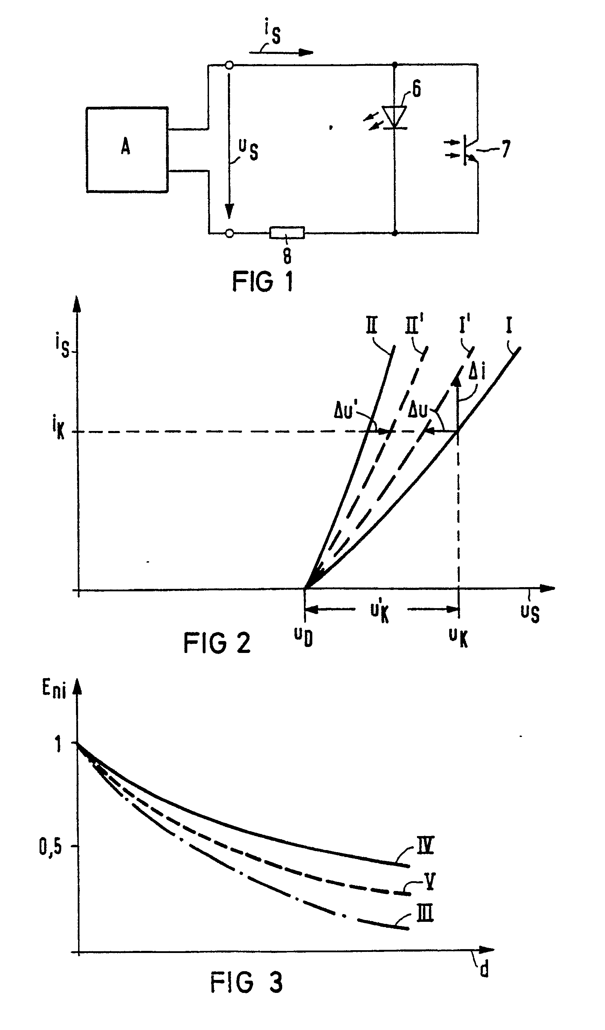

Eine derartige, zur Veranschaulichung der Problemstellung in Fig. 1 schematisch dargestellte Meßvorrichtung ist aus der DE-PS 31 52 963 bekannt. Dabei enthält die Meßsonde eine Kombination aus einer Leuchtdiode 6 und einem Fototransistor 7, die derart parallel geschaltet sind, daß der Durchlaßstrom durch die Leuchtdiode 6 additiv überlagert wird von dem durch Lichteinwirkung verursachten Strom durch den Fototransistor. Der Widerstand 8 stellt den Widerstand der Zuleitung dar. Bei Ansteuerung der Meßsonde durch die Auswerteschaltung A mit einem konstanten Strom oder einer konstanten Spannung löst das vom Blut abhängig von seiner Sauerstoffsättigung reflektierte Licht im Fototransistor 7 einen Stromfluß aus, der eine Stromänderung bzw. Spannungsänderung an der Meßsonde bewirkt. Die durch Lichtreflexion erzeugte Spannungs- bzw. Stromänderung wird in einer Auswerteschaltung durch Vergleich des Meßsignals bei angesteuerter Leuchtdiode und Fototransistor mit einem Referenzsignal ermittelt.

Fig. 2 zeigt eine Strom-Spannungs-Kennlinie der Meßsonde nach Fig. 1. Dabei ist die Kennlinie, die sich ohne Strom durch den Fototransistor 7, d.h. ohne Reflexion ergibt, mit I bezeichnet. Mit Reflexion entsteht dann die Kennlinie I'.

Für die Messung ist die spezifische Empfindlichkeit $\bar{E}$ der Sonde als Funktion der Blutsauerstoffsättigung $S_{02}$ ein wichtiges Kriterium. Bei Betrieb mit konstantem Strom wird diese im folgenden als $E_i$ und bei Betrieb mit konstanter Spannung mit $E_u$ bezeichnet. Es ist also definiert:

- bei konstantem Sondenstrom: $i_s = i_k$

$$E_i = \frac{du_s}{dS_{02}} \qquad \left[\frac{mV}{\%}\right] \qquad (1)$$

- bei konstanter Sondenspannung: $U_S = U_K$

$$E_u = \frac{di_s}{dS_{02}} \qquad \left[\frac{mA}{\%}\right] \qquad (2)$$

wobei entsprechend Fig. 2 gilt:

$u_s$ [mV] = Spannung an der Sonde (Sensor + Zuleitung)

$i_s$ [mA] = Strom durch die Sonde (Sensor + Zuleitung)

$$S_{02} \left[\%\right] = \frac{HbO_2}{Hb + HbO_2}$$

Anteil des oxigenierten Hämoglobin (Hb) am Gesamthämoglobin (Hb + HbO$_2$) kurz: Blutsauerstoffsättigung

Berechnet man entsprechend Fig. 2 die spezifische Empfindlichkeit, so ergibt sich für $E_i$ und $E_u$ vereinfacht:

$$E_i = -i_K \cdot \frac{1}{L_D} \cdot \frac{1}{(1 + K)^2} \cdot \frac{dK}{dS_{02}} \qquad (3)$$

$$E_u = u_K' \cdot L_D \cdot \frac{1}{[1 + R_D L_D \cdot (1 + K)]^2} \cdot \frac{dK}{dS_{02}} \cdot (4)$$

hierbei sind:

$$R_D \, [\Omega] \quad = \text{Serienwiderstand (5) zur Leuchtdiode 6.}$$

$$L_D \left[\frac{1}{\Omega}\right] = \frac{1}{R_{LED} + R_D} \quad \text{Steigung der Strom-Spannungskennlinie der Leuchtdiode 6, bzw. des Sensors bei } K = 0$$

$$u_K{}' \, [V] = u_K - u_D = \text{Konstantspannung, wobei } u_D \text{ die Durchlaßspannung der Leuchtdiode 6 ist}$$

$$R_L \, [\Omega] \qquad \text{Zuleitungswiderstand zum Sensor}$$

$$K = \frac{1}{100} \cdot k \, [\%] \quad \text{optischer Koppelfaktor, abhängig von der Art der Fotoelemente, der Sensorgeometrie, Ablagerungen auf dem Sensor und der Sauerstoffsättigung}$$

$$\frac{dk}{dS_{02}} \qquad \text{Änderung des Koppelfaktors, abhängig von } S_{02}$$

Aus den Gleichungen (3) und (4) ist zu ersehen, daß die Empfindlichkeit der Sonde in beiden Betriebszuständen mit wachsendem Koppelfaktor K geringer wird, ein nur für diese zweipolige Ausführungsform der Reflexionssonde charakteristisches Verhalten.

Solange k < 10%, also K < 0,1 ist dieses Meßverhalten der Sonde unproblematisch. Grundsätzlich wird jedoch ein möglichst hoher Koppelfaktor angestrebt, um Strom zu sparen und eine hohe $S_{02}$-Empfindlichkeit zu erreichen, da ja K ~ $S_{02}$, so daß die Sonde vorteilhaft im Grenzbereich K ~ 0,1 arbeitet. Damit entsteht das Problem, daß Änderungen im Reflexionsraum, die K erhöhen, zu einer deutlichen Verringerung der Sondenempfindlichkeit führen. Zu einer derartigen Veränderung kommt es bei Blut- oder Gewebeablagerungen an der Sonde, mit denen bei intrakardialer Implantation gerechnet werden muß. Die Ablagerung verringert die Empfindlichkeit der Sonde dann nicht nur, indem sie den Übergang des Lichts zwischen Sensor und Blut und damit den Faktor

$$\frac{dK}{dS_{02}}$$

dämpft, sondern auch durch wechselndes K infolge der Lichtreflexion an der nicht $S_{02}$-abhängigen Ablagerung, im folgenden als "Nullreflexion" bezeichnet.

Die Abnahme der Empfindlichkeit kann auch dadurch erklärt werden, daß bei der bekannten Anordnung der durch den Lichtsender fließende Sendestrom durch den durch den Lichtempfänger fließenden Empfangsstrom reduziert wird, d.h. also, daß bei steigender Nullreflexion weniger Sendestrom zur Verfügung steht.

In Fig. 3 ist für Konstantstrombetrieb die normierte Empfindlichkeit $E_{ni}$ in Abhängigkeit von Ablagerungen der Dicke d an der Meßsonde dargestellt. Dabei ist die Empfindlichkeit $E_{ni}$ so normiert, daß sich ohne Ablagerungen jeweils der Wert 1 ergibt. Für den genannten Stand der Technik gilt die Kennlinie III. Dabei ist deutlich die starke Abnahme der Empfindlichkeit mit zunehmenden Ablagerungen sichtbar. Diese Abnahme der Empfindlichkeit schafft Probleme bezüglich des Auflösungsbereichs des Meßverstärkers. Um den Schaltungsaufwand nicht zu hoch zu treiben, können nämlich keine Meßverstärker mit allzu hohem Dynamikbereich eingesetzt werden.

Aufgabe der Erfindung ist es daher, eine Meßvorrichtung der eingangs genannten Art so auszugestalten, daß die Abnahme der Empfindlichkeit bei Ablagerungen an der Meßsonde geringer wird.

Diese Aufgabe wird in einer ersten Lösungsvariante dadurch gelöst, daß parallel zum Lichtsender eine Transistorschaltung angeordnet ist, die vom Lichtempfänger so gesteuert wird, daß ihre Stromaufnahme mit zunehmendem Lichteinfall abnimmt.

Nullreflexionen bewirken damit eine Abnahme der Steigung der Strom/Spannungskennlinie der Meßsonde, also eine zunehmende Empfindlichkeit. Die Empfindlichkeit sinkt daher bei Ablagerungen nur noch aufgrund von Dämpfungen auf dem Lichtübertragungsweg, wobei sich durch Ablagerungen eine kleinere Verringerung der Empfindlichkeit als beim Stand der Technik ergibt.

In einer weiteren Lösung ist in Serie zum Lichtsender ein erster Widerstand angeordnet, dem ein in Abhängigkeit von dem vom Blut reflektierten Licht steuerbares Element parallel geschaltet ist. Damit wird verhindert, daß mit steigender Reflexion der Sendestrom durch den Empfangsstrom verringert wird, so daß sich bei Ablagerungen ebenfalls eine kleinere Verringerung der Empfindlichkeit ergibt.

Der Gedanke, zur Lösung der Aufgabe eine Verringerung des Sendestroms durch den Empfangsstrom zu vermeiden, ist beiden Lösungswegen gemeinsam.

In einer vorteilhaften Ausführungsform ist in Serie zum Lichtempfänger ein zweiter Widerstand angeordnet und parallel zu dieser Serienschaltung ein Transistor ange ordnet, dessen Steueranschluß mit dem Verbindungspunkt des zweiten Widerstands und des Lichtempfängers verbunden ist. Das bedeutet, daß dieser Verbindungspunkt bei Verwendung eines Bipolar-Transistors an dessen Basis und bei Verwendung eines Feldeffekttransistors an dessen Gate angeschlossen ist.

Vorteilhafterweise ist in Serie zum Lichtsender ein erster Widerstand angeordnet. Damit wird die Strom-Spannungskennlinie im Konstantstrombetrieb insgesamt flacher und die Anordnung empfindlicher.

Eine einfache Schaltung nach der zweiten Lösungsvariante erhält man, wenn in Serie zum Lichtempfänger ein zweiter Widerstand angeordnet ist und wenn das steuerbare Element ein Transistor ist, dessen Basis mit dem Verbindungspunkt von Lichtempfänger und zweitem Widerstand verbunden ist.

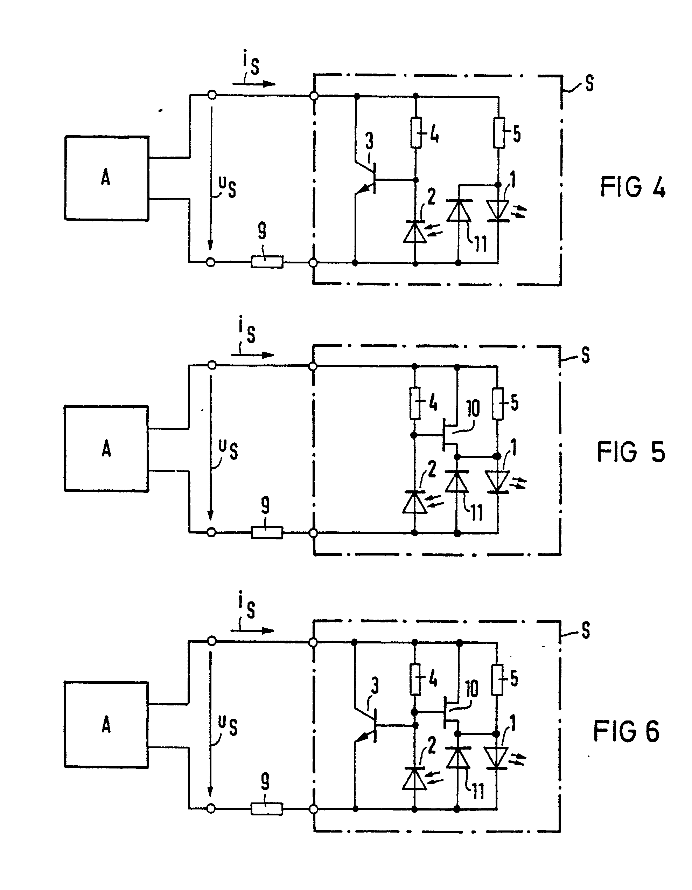

Ein Ausführungsbeispiel der Erfindung nach der ersten Lösungsvariante wird nachfolgend anhand der Fig. 4 näher erläutert. Da es sich in der Praxis als vorteilhaft erwiesen hat, die Meßsonde mit Konstantstrom anzusteuern, weil hierbei der Zuleitungswiderstand $R_L$ (9) ohne Einfluß auf die Empfindlichkeit ist, wird im folgenden die Schaltung mit Serienwiderstand $R_D$ (5) besprochen. Dabei enthält die Meßsonde S die Parallelschaltung eines Transistors 3, einer Serienschaltung eines Widerstands 4 und einer lichtempfindlichen Diode 2 als Lichtempfänger sowie eines Widerstands 5 und einer Leuchtdiode 1 als Lichtsender. Die Meßsonde S wird von einer Auswerteschaltung A mit einem konstanten Strom $i_s$ beaufschlagt. Hierbei wird mit wachsender Reflexion des Lichtes am Blut in Abhängigkeit von dessen Sauerstoffsättigung der Stromfluß durch die lichtempfindliche Diode 2 größer. Damit wird der über den Widerstand 4 der Basis des Transistors 3 zugeführte Strom reduziert, so daß dessen Leitfähigkeit abnimmt. Wenn beispielsweise die Meßsonde S ohne Reflexion die in Fig. 2 mit II bezeichnete Strom-Spannungskennlinie hat, so weist sie mit Reflexion z.B. die mit II′ bezeichnete Kennlinie auf. Bei Ansteuerung mit konstantem Strom $i_s = i_k$ nimmt also die Spannung um $\Delta U$ zu. Damit wird, wie erwünscht, die Strom-Spannungskennlinie $\Delta I / \Delta U$ flacher und damit die Anordnung empfindlicher.

In Fig. 3 ist die Abhängigkeit der normierten Empfindlichkeit $E_{ni}$ in Abhängigkeit von Ablagerungen d mit IV bezeichnet. Die Abnahme der Empfindlichkeit $E_{ni}$ bei Ablagerungen ist nunmehr lediglich noch durch Dämpfungen auf dem Übertragungsweg bedingt und wesentlich geringer als beim Stand der Technik.

Eine Erhöhung der Empfindlichkeit der Anordnung ist auch mit dem Widerstand 5 erreicht, da dieser ebenfalls eine Abflachung der Kennlinie $\Delta I / \Delta U$ bewirkt.

Ein Ausführungsbeispiel der Erfindung nach der zweiten Lösungsvariante ist in Figur 5 dargestellt. Dabei sind in der Meßsonde S ebenfalls die Reihenschaltung eines Widerstandes 4 und einer lichtempfindlichen Diode 2 sowie eines Widerstandes 5 und einer Leuchtdiode 1 parallel geschaltet. Außerdem ist dem Widerstand 5 ein Feldeffekttransistor 10 parallel geschaltet, dessen Gate mit dem Verbindungspunkt von Widerstand 4 und lichtempfindlicher Diode 2 verbunden ist. Schließlich ist der Leuchtdiode 1 eine Diode 11 parallel geschaltet, deren Leitrichtung entgegengesetzt zur Leitrichtung der Leuchtdiode ist.

Wenn bei dieser Schaltung die Lichtreflexion am Blut zunimmt, so steigt die Stromaufnahme der lichtempfindlichen Diode 2. Damit wird der Widerstand des Feldeffekt transistors 10 verringert, so daß insgesamt der durch die Leuchtdiode 1 fließende Strom nicht - wie beim Stand der Technik - durch den durch die lichtempfindliche Diode 2 fließenden Empfangsstrom verringert wird. Damit ergibt sich - wie bei der ersten Lösungsvariante- bei Ablagerungen an der Sonde und damit steigender Nullreflexion ein

EP 0 220 549 B1

neutraleres Verhalten der Empfindlichkeit als beim Stand der Technik. Die Änderung der normierten Empfindlichkeit $E_n$ in Abhängigkeit von Ablagerungen d ist in Figur 3 gestrichelt eingezeichnet und mit V bezeichnet. Man erkennt, daß die Abnahme der Empfindlichkeit deutlich geringer ist als bei der mit III charakterisierten bekannten Anordnung, allerdings etwas größer als bei der mit IV charakterisierten ersten Lösungsvariante.

Die in den Schaltungen nach den Figuren 4 und 5 noch eingezeichnete Diode 11 dient zur Referenzmessung. Zur Referenzmessung wird die Stromrichtung umgekehrt, so daß durch die Sendediode 1 kein Strom mehr fließt. Damit erhält man eine Meßgröße, die vom reflektierten Licht unabhängig ist und nur vom Zuleitungswiderstand 9 und von der Temperatur der Meßsonde abhängt. Diese Referenzmessung kann zur Kompensation dieser Einflüsse auf die Messung herangezogen werden.

Das Ausführungsbeispiel nach Fig. 6 stellt eine Kombination der Ausführungsbeispiele nach den Fig. 4 und 5 dar, d.h. im Vergleich zur Schaltung nach Fig. 5 ist parallel zur Meßanordnung wie beim Ausführungsbeispiel nach Fig. 4 ein Transistor 3 geschaltet. Die Leitfähigkeit des Transistors 3 wird ebenfalls kleiner, wenn der Stromfluß durch die lichtempfindliche Diode 2 größer wird. Bei dieser Ausführungsform nimmt die Empfindlichkeit bei Ablagerungen an der Meßsonde noch weniger ab.

**Ansprüche**

1. Meßvorrichtung zur intrakardialen Erfassung der Blutsauerstoffsättigung zur Frequenzregelung eines Herzschrittmachers mit einer Meßsonde (S), die einen Lichtsender (1) und einen Lichtempfänger (2) enthält, wobei der Lichtempfänger (2) das vom Lichtsender (1) ausgesandte und vom Blut reflektierte Licht empfängt und wobei der Lichtsender (1) und der Lichtempfänger (2) über zwei gemeinsame Leitungen an eine Auswerteschaltung (A) angeschlossen sind, die die Meßsonde (S) mit einem konstanten Strom oder einer konstanten Spannung beaufschlagt,
   **dadurch gekennzeichnet,**
   daß parallel zum Lichtsender (1) eine Transistorschaltung (3) angeordnet ist, die vom Lichtempfänger (2) so gesteuert wird, daß ihre Stromaufnahme mit zunehmendem Lichteinfall abnimmt.

2. Meßvorrichtung zur intrakardialen Erfassung der Blutsauerstoffsättigung zur Frequenzregelung eines Herzschrittmachers mit einer Meßsonde (S), die einen Lichtsender (1) und einen Lichtempfänger (2) enthält, wobei der Lichtempfänger (2) das vom Lichtsender (1) ausgesandte und vom Blut reflektierte Licht empfängt und wobei der Lichtsender (1) und der Lichtempfänger (2) über zwei gemeinsame Leitungen an eine Auswerteschaltung (A) angeschlossen sind, die die Meßsonde (S) mit einem konstanten Strom oder einer konstanten Spannung beaufschlagt,
   **dadurch gekennzeichnet,**
   daß in Serie zum Lichtsender (1) ein erster Widerstand (5) angeordnet ist, dem ein in Abhängigkeit von dem vom Blut reflektierten Licht steuerbares Element (10) parallel geschaltet ist.

3. Meßvorrichtung nach Anspruch 1,
   **dadurch gekennzeichnet,**
   daß in Serie zum Lichtempfänger (2) ein zweiter Widerstand (4) angeordnet und daß parallel zu dieser Serienschaltung ein Transistor (3) angeordnet ist, dessen Steueranschluß mit dem Verbindungspunkt des zweiten Widerstands (4) und des Lichtempfängers (2) verbunden ist.

4. Meßvorrichtung nach Anspruch 3,
   **dadurch gekennzeichnet,**
   daß in Serie zum Lichtsender (1) ein erster Widerstand (5) angeordnet ist.

5. Meßvorrichtung nach Anspruch 2,
   **dadurch gekennzeichnet,**
   daß in Serie zum Lichtempfänger (2) ein zweiter Widerstand (4) angeordnet ist und daß das steuerbare Element (10) ein Transistor ist, dessen Basis mit dem Verbindungspunkt von Lichtempfänger (2) und zweitem Widerstand (4) verbunden ist.

**Claims**

5

1. Measuring device for the intracardiac detection of blood oxygen concentration for the frequency control of a heart pacemaker, having a measuring probe (S) containing a light transmitter (1) and a light receiver (2), the light receiver (2) receiving light from the light transmitter (1) reflected by the blood, and the light transmitter (1) and the light receiver (2) being connected by means of two common leads to an evaluation circuit (A) which supplies the measuring probe (S) with a constant current or with constant voltage,
characterised in that
a transistor circuit (3), which is controlled by the light receiver (2) so that its current consumption decreases as the light incidence increases, is arranged in parallel with the light transmitter (1).

2. Measuring device for intracardial detection of blood oxygen concentration for the frequency control of a heart pacemaker, having a measuring probe (S) containing a light transmitter (1) and a light receiver (2), the light receiver (2) receiving light from the light transmitter (1) reflected by the blood, and the light transmitter (1) and the light receiver (2) being connected by means of two common leads to an evaluation circuit (A) which supplies the measuring probe (S) with a constant current or with constant voltage,
characterised in that
a first resistor (5) is connected in series with the light transmitter (1), a device (10) controllable in dependence on the light reflected by the blood being connected in parallel with this first resistor.

3. Measuring device according to claim 1, characterised in that a second resistor (4) is connected in series with the light receiver (2), and a transistor (3), whose control terminal is connected to the junction of the second resistor (4) and of the light receiver (2), is arranged in parallel to this series connection.

4. Measuring device according to claim 3, characterised in that a first resistor (5) is arranged in series with the light transmitter (2).

5. Measuring device according to claim 2, characterised in that a second resistor (4) is arranged in series with the light receiver (2) and in that the controllable element (10) is a transistor whose base is connected to the junction of the light receiver (2) and the second resistor (4).


**Revendications**

1. Dispositif de mesure servant à déterminer, de façon intracardiaque, la saturation du sang en oxygène en vue de régler la fréquence d'un stimulateur cardiaque, et comportant une sonde de mesure (S), qui comporte une source de lumière (1) et un récepteur de lumière (2), et dans lequel le récepteur de lumière (2) reçoit la lumière émise par la source de lumière (1) et réfléchie par le sang, et la source de lumière (1) et le récepteur de lumière (2) sont raccordés au moyen de deux conducteurs communs à un circuit d'évaluation (A), qui charge la sonde de mesure (S) avec un courant constant ou avec une tension constante, caractérisé par le fait qu'en parallèle avec la source de lumière (1) est branché un circuit à transistor (3), qui est commandé par le récepteur de lumière (2) de telle sorte que son absorption de courant diminue lors d'une augmentation de la lumière incidente.

2. Dispositif de mesure servant à déterminer, de façon intracardiaque, la saturation du sang en oxygène en vue de régler la fréquence d'un stimulateur cardiaque, et comportant une sonde de mesure (S), qui contient une source de lumière (1) et un récepteur de lumière (2), et dans lequel le récepteur de lumière (2) reçoit la lumière émise par la source de lumière (1) et réfléchie par le sang, et la source de lumière (1) et le récepteur de lumière (2) sont raccordés au moyen de deux conducteurs communs à un circuit d'évaluation (A), qui charge la sonde de mesure (S) avec un courant constant ou avec une tension constante, caractérisé par le fait qu'en série avec la source de lumière (1) est branchée une première résistance (5), en parallèle avec laquelle est branché un élément (10) commandable en fonction de la lumière réfléchie par le sang.

3. Dispositif de mesure suivant la revendication 1, caractérisé par le fait qu'une seconde résistance (4) est branchée en série avec le récepteur de lumière (2) et qu'en parallèle avec ce circuit série est branché un transistor (3), dont la borne de commande est raccordée au point de jonction entre la seconde résistance (4) et le récepteur de lumière (2).

4. Dispositif de mesure suivant la revendication 3, caractérisé par le fait qu'une première résistance (5) est branchée en série avec la source de lumière (1).

5. Dispositif de mesure suivant la revendication 2, caractérisé par le fait qu'une seconde résistance (4) est branchée en série avec le récepteur de lumière (2) et que l'élément commandable (10) est un transistor, dont la base est reliée au point de jonction entre le récepteur de lumière (2) et la seconde résistance (4).

FIG 1

FIG 2

FIG 3

FIG 4

FIG 5

FIG 6